# EUROPEAN PATENT SPECIFICATION

(11) **EP 1 941 888 B1**
(45) Date of publication and mention of the grant of the patent: **14.06.2017**
(21) Application number: 06798309.8
(22) Date of filing: 25.09.2006
(51) Int. Cl.: A23L 2/00, A61K 35/02, A61K 33/00, A61K 33/06, A61P 31/04, A23L 33/16

(54) **AGENT FOR INHIBITING THE PROLIFERATION AND MIGRATION OF HELICOBACTER PYLORI**
MITTEL ZUR HEMMUNG DER PROLIFERATION UND MIGRATION VON HELICOBACTER PYLORI
AGENT POUR L'INHIBITION DE LA PROLIFÉRATION ET DE LA MIGRATION DE HELICOBACTER PYLORI

(30) Priority: 26.09.2005 JP 2005278540
(43) Date of publication of application: 09.07.2008
(73) Proprietor: Ako Kasei Co., Ltd., Ako-city, Hyogo 678-0172 (JP); National University Corporation Kochi University, Kochi-shi, Kochi 780-8520 (JP)
(72) Inventor: TAKEUCHI, Hiroaki, Kochi-shi, Kochi 781-8123 (JP); IKEGAMI, Yoshinari, Ako-shi, Hyogo 678-0172 (JP); YASUKAWA, Takeshi, Ako-shi, Hyogo 678-0172 (JP); NAKAGAWA, Koji, Ako-shi, Hyogo 678-0172 (JP)
(74) Representative: Vossius & Partner Patentanwälte Rechtsanwälte mbB
(86) International application number: PCT/JP2006/318983
(87) International publication number: WO 2007/034948

(56) References cited:
- EP-A1- 1 170 013
- WO-A1-02/40051
- WO-A1-95/32720
- WO-A1-98/22117
- WO-A1-99/64420
- DE-A1- 19 544 414
- JP-A- 10 182 424
- JP-A- 2001 198 575
- JP-A- 2001 224 326
- JP-A- 2001 314 174
- JP-A- 2001 321 062
- JP-A- 2002 125 615
- JP-A- 2002 191 331
- JP-A- 2002 302 401
- JP-A- 2002 306 118
- JP-A- 2003 159 031
- JP-A- 2003 306 405
- JP-A- 2005 151 981
- US-A- 5 834 002
- YASUKAWA T. ET AL.: 'Kaiyo Shinsosui yori Chosei shita Ko-Mineral Water no Helicobacter.Pylori ni Taisuru Eikyo' JAPANESE JOURNAL OF CLINICAL LABORATORY AUTOMATION vol. 30, no. 4, 01 September 2005, page 532, LECTURE TITLE 150, XP003010776

## Description

### Technical Field

The present invention relates to an agent for inhibiting the proliferation and migration of Helicobacter pylori strains that are considered a cause of gastric ulcer, duodenal ulcer, stomach cancer, etc.

More specifically, the present invention relates to an agent for inhibiting the proliferation and migration of Helicobacter pylori strains in a manner not producing resistant bacteria to, or harmful side effects from treatment drugs of, Helicobacter pylori strains that are indigenous to the host.

For clarification purposes, in the context of the present invention an agent for inhibiting the proliferation and migration of Helicobacter pylori strains refers to both an agent for inhibiting the proliferation of Helicobacter pylori strains and an agent for inhibiting the active migration of Helicobacter pylori strains.

Pumping of deep sea water began in Japan in 1989 off the coast of Cape Muroto in Kochi Prefecture using a deep sea water collection facility constructed on land. Today, deep sea water is collected in various parts of Japan including Toyama Prefecture and Okinawa Prefecture. It is well-known that deep sea water has various beneficial characteristics such as containing a lot of nutrients, being clean, and remaining stable at low temperature. Accordingly, many products that use deep sea water have been developed in recent years. Among others, there is a trend of active utilization of deep sea water in the food industry in the production of drinking water, processed seafood products, fermented products, etc., and a number of products using deep sea water are now available on the market.

Many of these products are drawing the attention as offering the benefits of deep sea water such as cleanliness and high mineral content, and utilized as a pure material used in product manufacturing or a source of quality minerals.

On the other hand, medical and scientific studies are gradually finding the functions and mechanisms embodied in the human body by drinking water that uses deep sea water and thereby contains a lot of minerals. Although deep sea water is expected to offer numerous benefits, such as regulating the actions of the intestines, having positive effects on the immune strength of the body, and preventing lifestyle-related diseases, the specific actions and effects of deep sea water including those mentioned above are not yet understood fully.

On the other hand, Helicobacter pylori strains infect and colonize in the human stomach and are reported to have a hand in the development of diseases of the upper gastrointestinal tracts as well as autoimmune diseases, acute coronary diseases, and lifestyle-related diseases, among others. In particular, it is a well-known fact that Helicobacter pylori strains are closely related to the causes of gastric ulcer, duodenal ulcer, stomach cancer, etc.

For this reason, various therapies are used to remove Helicobacter pylori strains, such as those using various antibacterial agents, proton pump inhibitors (PPIs) that inhibit secretion of gastric acid, and antibiotics. As a specific example, a local oral agent that contains a bismuth compound, such as bismuth citrate, to kill Helicobacter pylori strains in the oral cavity is disclosed in Patent Literature 1 (Japanese Patent Laid-open No. Hei 8-20543). Also, Patent Literature 2 (Japanese Patent Laid-open No. Hei 8-48629) and Patent Literature 3 (Japanese Patent Laid-open No. Hei 9-208578) disclose anti-pylori agents exhibiting excellent antibacterial activity against Helicobacter pylori strains, where the effective ingredient is pyridone carboxylic acid, its ester, or salt thereof.

In addition to the above, Patent Literature 4 (Japanese Patent Laid-open No. Hei 9-295938) discloses an ulcer treatment drug, whose effective ingredients include a carbapenem compound or its pharmacologically acceptable form of salt having strong antibacterial activity against Helicobacter pylori strains that colonize and grow in the internal membranes of the stomach, for use as a drug to treat digestive ulcers and chronic superficial gastritis as well as prevent their recurrence.

In addition, Patent Literature 5 (Japanese Patent Laid-open No. 2000-63280) and Patent Literature 6 (Japanese Patent Laid-open No. Hei 10-109942) also disclose drugs that combine coptis root, cork tree bark or other galenical preparation in powder or extract form having antibacterial activity against Helicobacter pylori strains, as an effective medicinal composition to treat or prevent gastrointestinal diseases by effectively inhibiting the proliferation of, or removing and discharging, Helicobacter pylori strains that are considered a cause of the so-called digestive ulcers such as gastritis, gastric ulcer and duodenal ulcer, with at least one ingredient selected from histamine H₂ receptor antagonists, proton pump inhibitors, gastric-mucosa protective drugs for gastritis and digestive ulcers, gastric anti-acid drugs, and anti-diarrheal drugs.

Furthermore, Patent Literature 7 (Japanese Patent Laid-open No. Hei 11-180888) discloses a polyphenol obtained from fruits of rosaceous plants as an effective ingredient for use in drugs, anti-infection agents, or food, having an antibacterial action against Helicobacter pylori strains, while Patent Literature 8 (Japanese Patent Laid-open No. Hei 11-292788) discloses a Fe-bonded lactoferrin for use in anti-infection agents, drugs or beverages/food. Similarly, Patent Literature 9 (Japanese Patent Laid-open No. 2002-68992) discloses a beverage/food or food additive containing tea polyphenol as an effective ingredient along with a proton inhibitor, while Patent Literature 10 (Japanese Patent Laid-open No. 2005-68014) discloses an antibacterial agent against Helicobacter pylori strains, constituted by one or two or more extracts selected from the group that includes Apocynum venetum L., Vaccinium myrtillus L. and Acanthopanax senticosus Harms.

However, the types of Helicobacter pylori strains present in the body are different from one host to another, and accordingly the effects of general antibacterial agents are not clear and cases of unsuccessful removal of Helicobacter pylori due to emergence of resistant bacteria and side effects are increasing.

For the aforementioned reasons, a number of simple methods for detecting and identifying Helicobacter pylori strains have been reported, including: an infection diagnosis probe useful in the detection and identification of Helicobacter pylori strains themselves, as disclosed in Patent Literature 11 (Japanese Patent Laid-open No. Hei 10-33179); a real-time infection inspection method based on the principles of "analysis of expired gas using ¹³C labeled urea," as disclosed in Patent Literature 12 (Japanese Patent Laid-open No. Hei 10-87512; a primer based on Helicobacter pylori strain genes and a method for examining clarithromycin resistance of Helicobacter pylori strains, as disclosed in Patent Literature 13 (Japanese Patent Laid-open No. Hei 10-286099) and Patent Literature 14 (Japanese Patent Laid-open No. 2001-321197); and a detection method using an enzyme urease derived from Helicobacter pylori strains, as disclosed in Patent Literature 15 (Japanese Patent Laid-open No. Hei 11-318490). However, utilization of deep sea water in the inhibition of proliferation or migration of Helicobacter pylori strains has not been reported to date.

Patent Literature 16 discloses a chewing gum composition containing a water soluble bulk portion, a water insoluble chewing gum base portion, a flavoring agent, and bismuth-containing compounds.

Patent Literature 17 relates to compositions and methods for the symptomatic treatment of gastrointestinal disease by pathogenic infection.

Patent Literature 18 discloses a composition including colostrum or a colostrum-derived product and hyperimmune milk (HIM) or a hyperimmune milk-derived product.

Patent Literature 19 relates to β-carboline compounds.

Patent Literature 20 relates to methods and compositions for treating a gastrointestinal disorder caused or mediated by *Helicobacter pylori.*

Patent Literature 21 discloses drugs, foods, drinks and feeds containing a cocoa component.

Patent Literature 22 refers to tropolone substituted bismuth compounds.

Patent Literature 23 relates to a freshening liquid and a method for producing the same.

Patent Literature 24 refers to a method for producing a preservation property-improving agent for fresh food.

Patent Literature 25 discloses a bath preparation.

Patent Literature 26 refers to an antibacterial agent consisting of weakly alkaline aqueous solution obtained by electrolyzing seawater and a method for imparting antibacterial action to seawater.

Patent Literature 27 discloses a method for producing soft drinking water utilizing deep sea water.

Patent Literature 28 discloses a method for producing mineral-enriched food and the food.

Patent Literature 29 refers to bread using deep water as raw material.

Patent Literature 30 discloses liquid salt for food.

Patent Literature 31 refers to a mineral replenishing material derived from deep ocean water.

Patent Literature 32 relates to a method for producing health salt from ocean deep water and a device thereof.

Patent Literature 33 discloses a bean cured using bittern derived from marine deep water and a method for producing the same.

Patent Literature 34 refers to a barley tea beverage.

Non-patent Literature 1 discusses effects of high-mineral water prepared from deep sea water on helicobacter Pylori.

Patent Literature 1: Japanese Patent Laid-open No. Hei 8-20543
Patent Literature 2: Japanese Patent Laid-open No. Hei 8-48629
Patent Literature 3: Japanese Patent Laid-open No. Hei 9-208578
Patent Literature 4: Japanese Patent Laid-open No. Hei 9-295938
Patent Literature 5: Japanese Patent Laid-open No. 2000-63280
Patent Literature 6: Japanese Patent Laid-open No. Hei 10-109942
Patent Literature 7: Japanese Patent Laid-open No. Hei 11-180888
Patent Literature 8: Japanese Patent Laid-open No. Hei 11-292788
Patent Literature 9: Japanese Patent Laid-open No. 2002-68992
Patent Literature 10: Japanese Patent Laid-open No. 2005-68014
Patent Literature 11: Japanese Patent Laid-open No. Hei 10-33179
Patent Literature 12: Japanese Patent Laid-open No. Hei 10-87512
Patent Literature 13: Japanese Patent Laid-open No. Hei 10-286099
Patent Literature 14: Japanese Patent Laid-open No. 2001-321197
Patent Literature 15: Japanese Patent Laid-open No. Hei 11-318490
Patent Literature 16: US 5 834 002
Patent Literature 17: WO 95/32720
Patent Literature 18: WO 02/40051
Patent Literature 19: WO 99/64420
Patent Literature 20: WO 98/22117
Patent Literature 21: EP 1 170 013
Patent Literature 22: DE 195 44 414
Patent Literature 23: JP 2002 302401
Patent Literature 24: JP 2003 306405
Patent Literature 25 JP 10 182424
Patent Literature 26: JP 2001 198575
Patent Literature 27: JP 2002 191331
Patent Literature 28: JP 2001 314174
Patent Literature 29: JP 2001 321062
Patent Literature 30: JP 2002 125615
Patent Literature 31: JP 2003 159031
Patent Literature 32: JP 2002 306118
Patent Literature 33: JP 2001 224326
Patent Literature 34: JP 2005 151981
Non-patent Literature 1: T. Yasukawa et al., Japanese Journal of Clinical Laboratory Automation, 30(4), 2005, p. 532.

### Summary of the Invention

### Problems to Be Solved by the Invention

As mentioned above, methods for preventing or treating gastrointestinal diseases are being sought, which are able to accommodate the diversity of Helicobacter pylori strains closely related to the causes of gastric ulcer, duodenal ulcer, stomach cancer, etc., and thereby suppress side effects of antibacterial agents and emergence of resistant bacteria. In the meantime, inhibition of proliferation and migration of Helicobacter pylori strains itself provides a means for prevention and treatment of diseases caused by Helicobacter pylori strains. When prevention is considered, it is safer for the health of human body and also provides an easier method of intake to continuously ingest as part of meals a food matter having the effect of inhibiting the proliferation and migration of Helicobacter pylori strains, instead of using a drug regularly.

In light of the above, it is an object of the present invention to achieve inhibition of proliferation and migration ofHelicobacter pylori strains through utilization of inexpensive materials, such as drinking water and other substances we consume daily, because such is an ideal way to inhibit the proliferation and migration of Helicoliacter pylori strains.

It is another object of the present invention to establish a technology for inhibiting the proliferation and migration of Helicobacter pylori strains that colonize in and infect the human stomach and are also reported to have a hand in diseases of the upper gastrointestinal tracts as well as autoimmune diseases, acute coronary diseases, and lifestyle-related diseases, among others, by focusing on deep sea water, which is rich in nutrients and minerals and inexpensive and therefore used widely in the food industry for use in the production of drinking water, processed seafood products, fermented products, etc., because deep sea water can be consumed easily in everyday life as drinking water.

### Means for Solving the Problems

In light of the above, the present invention establishes a technology for inhibiting the proliferation and migration of Helicobacter pylori strains by utilizing mineral-rich water. To be specific, the present invention represents a liquid (agent) for inhibiting the proliferation and migration of Helicobacter pylori strains which contains at least one or more of magnesium, calcium, sodium and potassium, based on the results of examination with focus on magnesium, calcium, sodium and potassium that exist in particularly high quantities in deep sea water among the various minerals contained in deep sea water, and also with focus on liquids (agents) that can be used as a way to ingest these materials easily in everyday life, and consequently the present invention establishes a technology for inhibiting the proliferation and migration of Helicobacter pylori strains by utilizing mineral-rich water derived from deep sea having a mineral content per kilogram of 5,900 mg or more. The minerals examined in connection with the present invention are derived from deep sea water subject to minimal contamination by harmful substances, etc., and thus can be used favorably as materials for beverages and food. However, it is not necessary to limit the source of these minerals to deep sea water.

If the mineral content per kilogram is 40 mg or more, proliferation and migration of Helicobacter pylori strains can be inhibited.

If the mineral content per kilogram is 5,900 mg or more, proliferation and migration of all Helicobacter pylori strains can be inhibited.

### Effects of the Invention

The greatest benefit of the present invention is that by consuming mineral-rich water obtained from deep sea water that is conveniently available, proliferation and migration of Helicobacter pylori strains indigenous to the host can be inhibited to prevent and treat diseases of the upper gastrointestinal tracts, among others, without being concerned about emergence of resistant bacteria and side effects of treatment drugs.

### Brief Description of the Drawings

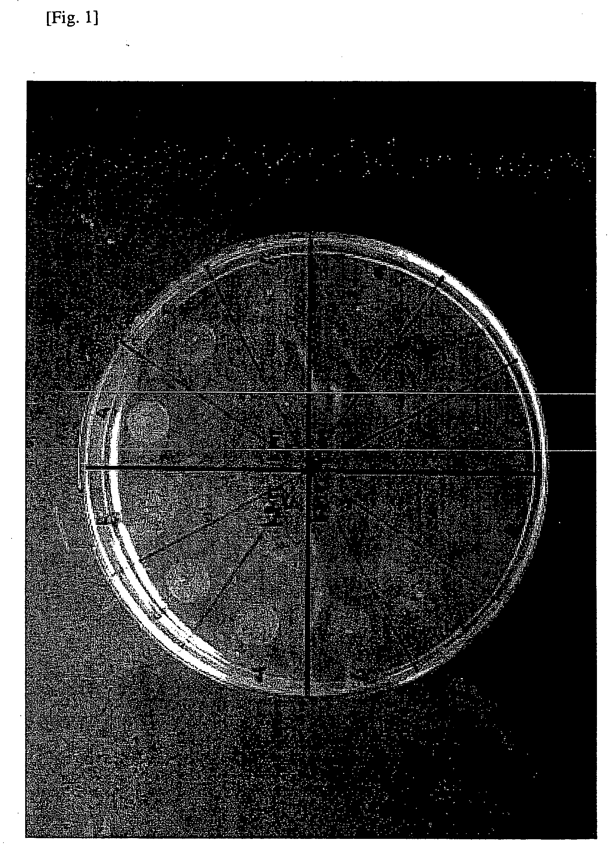

[Fig. 1] condition of colonies that grew in an agar culture medium

### Best Mode for Carrying Out the Invention

### [Method for Testing Inhibition of Proliferation of Helicobacter Pylori Strains]

The method for testing the inhibition of proliferation of Helicobacter pylori strains used in the present invention is explained below.

Each type of Helicobacter pylori strains corresponding to approx. 1 platinum loop was taken from an agar culture medium (Table 1) on the second day of culture and suspended in 1 ml of 2.8% Brucella broth culture solution so that O.D.₆₀₀ became 0.3.

The prepared solution was then diluted to 10⁻⁴, 10⁻⁵ and 10⁻⁶ times, respectively, using sterilized water, and each bacteria-suspended solution was dripped by 10 µl each into agar culture mediums (Table 3) containing mineral-rich waters A, B, C, D, E, F and G (test waters) prepared from deep sea water as shown in Table 2. Next, the culture mediums were cultured for three days in an environment of 37°C and 10% CO₂, after which the CFU (colony forming unit = viable bacterial cell count) of each culture medium was measured:
For your reference, Milli-Q water was used as a control water.

**[Table 1]**

| Composition of agar culture medium | |
|---|---|
| Composition | Content |
| Brucella Broth | 2.8 g |
| Milli-Q water | 90 ml |
| Agar powder | 1.4 g |
| Horse serum | 10 ml |
| Vancomycin solution (100 mg/ml) | 10 µl |

**[Table 2]**

| Compositions of mineral-rich waters (mg/kg) | | | | |
|---|---|---|---|---|
| Mineral-rich water / Ion type | Mg | Ca | Na | K |
| A | 100 | 200 | 50 | 50 |
| B | 150 | 150 | 50 | 50 |
| C | 200 | 70 | 65 | 65 |
| D | 400 | - | - | - |
| E | - | 400 | - | - |
| F | - | - | 400 | - |
| G | - | - | - | 400 |

**[Table 3]**

| Composition of agar culture medium | |
|---|---|
| Composition | Content |
| Brucella Broth | 2.8 g |
| Test water or control water | 90 ml |
| Agar powder | 1.4 g |
| Horse serum | 10 ml |
| Vancomycin solution (100 mg/ml) | 10 µl |

### [Helicobacter Pylori Strains Used]

In the test, mainly clinically isolated strains were used.
KMT series: Clinically isolated strain obtained from the Medical School of Kochi University NY series: Strain resistant to antibiotics (metronidazole and clarithromycin) (made in Japan) 26695: Clinically isolated strain analyzed in details to gene level (obtained from Europe)
NCTC11637: Clinically isolated strain (obtained from the U.S.)
HPK55: Clinically isolated strain (obtained in Japan)
HPK5510: Broken strain of cdrA gene (gene relating to cell division) of HPK5 prepared by means of gene recombination

### [Method for Evaluating Proliferation Inhibition]

The effects of inhibiting the proliferation of Helicobacter pylori strains in test waters A, B, C, D, E, F and G were determined based on the CFU (colony forming unit = viable bacterial cell count) of the control water being 100%.

By considering the variations resulting from the operations carried out in the test, test waters whose CFU was smaller than 80% of the CFU of the control water were considered effective. The test was conducted three times and if effectiveness was found in two test runs, the test water was judged effective in inhibiting Helicobacter pylori proliferation (indicated by ○).

### [Method for Testing Inhibition of Migration of Helicobacter Pylori Strains]

Using a sterilized toothpick, bacteria of each type were taken from agar culture mediums in which Helicobacter pylori strains had been cultured, and then transferred to Brucella broth agar culture mediums (Table 4) using mineral-rich waters A, B, C, D, E, F and G prepared from deep sea water and also using Milli-Q water as a control. Next, the culture mediums were cultured for three days in an environment of 37°C and 10% CO₂, after which the diameters of colonies in each culture medium were measured.

**[Table 4]**

| Composition of agar culture medium | |
|---|---|
| Composition | Content |
| Brucella Broth | 2.8 g |
| Test water or control water | 90 ml |
| Agar powder | 0.35 g |
| Horse serum | 10 ml |
| Vancomycin solution (100 mg/ml) | 10 µl |

### [Method for Evaluating Migration Inhibition]

In the evaluation, test waters whose colonies had a diameter smaller than the diameters of colonies in the control water were considered effective. The test was conducted three times and if effectiveness was found in two test runs, the test water was judged effective in inhibiting Helicobacter pylori migration (indicated by ⊙).

For your reference, colonies with a diameter less than 2 mm in the control were considered not sufficiently grown and not counted.

The present invention is explained below using examples with respect to test waters A, B, C, D, E, F and G. It should be noted, however, that mineral-rich waters prepared form deep sea water are not at all limited to these waters.

### Comparative Example 1

Table 5 shows the effects of test waters in inhibiting the proliferation of various types of Helicobacter pylori strains.

**[Table 5]**

| Effects of inhibiting proliferation of various types of Helicobacter pylori strains | | | | | | | | |
|---|---|---|---|---|---|---|---|---|
| No. | Bacteria name | A | B | C | D | E | F | G |
| 1 | KMT27 | ○ | ○ | ○ | ○ | ○ | | |
| 2 | KMT31 | | ○ | | ○ | ○ | | |
| 3 | KMT40 | | ○ | ○ | | ○ | ○ | |
| 4 | KMT44 | | ○ | | ○ | | | ○ |
| 5 | KMT45 | ○ | ○ | | ○ | | ○ | |
| 6 | KMT46 | | | ○ | ○ | ○ | | |
| 7 | KMT47 | ○ | ○ | | ○ | ○ | | |
| 8 | KMT50 | ○ | ○ | ○ | ○ | ○ | | |
| 9 | NY1 | | | | | | ○ | ○ |
| 10 | NY8 | | | ○ | ○ | | | |
| 11 | NY11 | | | | | | | |
| 12 | NY31 | | ○ | ○ | ○ | ○ | | |
| 13 | 26695 | | | | | | | |
| 14 | HPK5 | | | ○ | ○ | | ○ | ○ |
| 15 | HPKT510 | ○ | | | | ○ | | |
| 16 | 11637 | ○ | ○ | | ○ | | | |

As shown in Table 5, whether proliferation of a given type of bacterial strain was inhibited or not varied depending on the type of test water.

Also, it is evident from Table 5 that test water D inhibited the proliferation of many types of bacterial strains, where clearly the effect of inhibiting Helicobacter pylori proliferation varies according to the type of bacterial strain.

Based on the above results, it is found that by consuming mineral-rich water conveniently available and obtained from deep sea water having the effect of inhibiting the proliferation of various types of Helicobacter pylori strains, diseases of the upper gastrointestinal tracts, among others, can be prevented and treated without being concerned about emergence of resistant bacteria and side effects of treatment drugs.

### Comparative Example 2

Table 6 shows the effects of inhibiting the migration of various types of Helicobacter pylori strains cultured in agar culture mediums using mineral-rich waters obtained from deep sea water.

**[Table 6]**

| Effects of inhibiting migration of various types of Helicobacter pylori strains | | | | | | | | |
|---|---|---|---|---|---|---|---|---|
| No. | Bacteria name | A | B | C | D | E | F | G |
| 1 | KMT27 | | | | | | | |
| 2 | KMT31 | ⊙ | | | | ⊙ | | |
| 3 | KMT40 | | | | | | | |
| 4 | KMT44 | | ⊙ | ⊙ | ⊙ | ⊙ | | |
| 5 | KMT45 | | ⊙ | ⊙ | ⊙ | ⊙ | | |
| 6 | KMT46 | | ⊙ | ⊙ | ⊙ | ⊙ | | |
| 7 | KMT47 | | | | | | | |
| 8 | KMT50 | | | | | | | |
| 9 | NY1 | | | | | | | |
| 10 | NY8 | ⊙ | ⊙ | | | ⊙ | | |
| 11 | NY11 | ⊙ | ⊙ | | | ⊙ | | |
| 12 | NY31 | | | | | | | |
| 13 | 26695 | | | | | | | |
| 14 | HPK5 | | ⊙ | ⊙ | | ⊙ | | |
| 15 | HPKT510 | | | | | ⊙ | | |
| 16 | 11637 | | | | | | | |

As shown in Table 6, test waters B, C, D, and E are effective in inhibiting the migration of KMT44, 45 and 46 strains.

Based on the above results, it is found that by selecting proper mineral-rich waters, migration of Helicobacter pylori strains infecting and colonized in the host can be inhibited.

### Example 3

Starting from mineral-rich waters each having a total mineral content of 400 mg/kg, the total mineral content of each water was changed within a range of 20 to 11,800 mg/kg based on the percentages of ion contents shown in Table 7, and the effects of varying total mineral concentrations on the inhibition of proliferation of Helicobacter pylori strains were examined.

**[Table 7]**

| Percentages of ion contents in various mineral waters (%) | | | | |
|---|---|---|---|---|
| Mineral-rich water / Ion type | Mg | Ca | Na | K |
| A | 25 | 50 | 12.5 | 12.5 |
| B | 37.5 | 37.5 | 12.5 | 12.5 |
| C | 50 | 17.5 | 16.25 | 16.25 |
| D | 100 | 0 | 0 | 0 |
| E | 0 | 100 | 0 | 0 |
| F | 0 | 0 | 100 | 0 |
| G | 0 | 0 | 0 | 100 |

By considering the variations resulting from the operations carried out in the test, test waters whose CFU was smaller than 80% of the CFU of the control water were considered effective. The test was conducted three times and if effectiveness was found in two test runs, the test water was judged effective in inhibiting Helicobacter pylori proliferation (indicated by ○).

For your reference, the strains used in the test were those Helicobacter pylori strains indicated in 1 through 16.

Tables 8 through 15 show the results of the examination where the total mineral concentration was gradually raised from 20 to 11,800 mg/kg.

**[Table 8]**

| Total mineral concentration = 20 mg/kg | | | | | | | | |
|---|---|---|---|---|---|---|---|---|
| No. | Bacteria name | A | B | C | D | E | F | G |
| 1 | KMT27 | | | | | | | |
| 2 | KMT31 | | | | | | | |
| 3 | KMT40 | | | | | | | |
| 4 | KMT44 | | | | | | | |
| 5 | KMT45 | | | | | | | |
| 6 | KMT46 | | | | | | | |
| 7 | KMT47 | | | | | | | |
| 8 | KMT50 | | | | | | | |
| 9 | NY1 | | | | | | | |
| 10 | NY8 | | | | | | | |
| 11 | NY11 | | | | | | | |
| 12 | NY31 | | | | | | | |
| 13 | 26695 | | | | | | | |
| 14 | HPK5 | | | | | | | |
| 15 | HPKT510 | | | | | | | |
| 16 | 11637 | | | | | | | |

**[Table 9]**

| Total mineral concentration = 40 mg/kg | | | | | | | | |
|---|---|---|---|---|---|---|---|---|
| No. | Bacteria name | A | B | C | D | E | F | G |
| 1 | KMT27 | | | ○ | ○ | | | |
| 2 | KMT31 | | | | | | | |
| 3 | KMT40 | | | | | | | |
| 4 | KMT44 | | | | | | | |
| 5 | KMT45 | | | | | | | |
| 6 | KMT46 | | | | | | | |
| 7 | KMT47 | | | | | | | |
| 8 | KMT50 | | | | | | | |
| 9 | NY1 | | | | | | | |
| 10 | NY8 | | | | | | | |
| 11 | NY11 | | | | | | | |
| 12 | NY31 | | ○ | | ○ | | | |
| 13 | 26695 | | | | | | | |
| 14 | HPK5 | | | | | | | |
| 15 | HPKT510 | | | | | | | |
| 16 | 11637 | | | | | | | |

**[Table 10]**

| Total mineral concentration = 100 mg/kg | | | | | | | | |
|---|---|---|---|---|---|---|---|---|
| No. | Bacteria name | A | B | C | D | E | F | G |
| 1 | KMT27 | | | ○ | ○ | | | |
| 2 | KMT31 | | | | | | | |
| 3 | KMT40 | | | | | | ○ | |
| 4 | KMT44 | | | | | | | ○ |
| 5 | KMT45 | | | | | | | |
| 6 | KMT46 | | | | | | | |
| 7 | KMT47 | | | | | | | |
| 8 | KMT50 | | ○ | ○ | | | | |
| 9 | NY1 | | | | | | | |
| 10 | NY8 | | | | | | | |
| 11 | NY11 | | | | | | | |
| 12 | NY31 | | ○ | ○ | ○ | ○ | | |
| 13 | 26695 | | | | | | | |
| 14 | HPK5 | | | | | | | |
| 15 | HPKT510 | | | | | | | |
| 16 | 11637 | | | | | | | |

**[Table 11]**

| Total mineral concentration = 1,200 mg/kg | | | | | | | | |
|---|---|---|---|---|---|---|---|---|
| No. | Bacteria name | A | B | C | D | E | F | G |
| 1 | KMT27 | ○ | ○ | ○ | ○ | ○ | ○ | ○ |
| 2 | KMT31 | | ○ | ○ | ○ | ○ | | |
| 3 | KMT40 | | ○ | ○ | | ○ | ○ | |
| 4 | KMT44 | ○ | ○ | | ○ | | ○ | ○ |
| 5 | KMT45 | ○ | ○ | ○ | ○ | ○ | ○ | ○ |
| 6 | KMT46 | | | ○ | ○ | ○ | | |
| 7 | KMT47 | ○ | ○ | ○ | ○ | ○ | | |
| 8 | KMT50 | ○ | ○ | ○ | ○ | ○ | | |
| 9 | NY1 | | | | | | ○ | ○ |
| 10 | NY8 | | | ○ | ○ | | | |
| 11 | NY11 | | | | | | | |
| 12 | NY31 | | ○ | ○ | ○ | ○ | | |
| 13 | 26695 | | | | | | | |
| 14 | HPK5 | | | ○ | ○ | | ○ | ○ |
| 15 | HPKT510 | ○ | | | | ○ | | |
| 16 | 11637 | ○ | ○ | | ○ | | | |

**[Table 12]**

| Total mineral concentration = 2,500 mg/kg | | | | | | | | |
|---|---|---|---|---|---|---|---|---|
| No. | Bacteria name | A | B | C | D | E | F | G |
| 1 | KMT27 | ○ | ○ | ○ | ○ | ○ | ○ | ○ |
| 2 | KMT31 | ○ | ○ | ○ | ○ | ○ | | |
| 3 | KMT40 | | ○ | ○ | | ○ | ○ | |
| 4 | KMT44 | ○ | ○ | | ○ | | ○ | ○ |
| 5 | KMT45 | ○ | ○ | ○ | ○ | ○ | ○ | ○ |
| 6 | KMT46 | ○ | ○ | ○ | ○ | ○ | ○ | ○ |
| 7 | KMT47 | ○ | ○ | ○ | ○ | ○ | ○ | ○ |
| 8 | KMT50 | ○ | ○ | ○ | ○ | ○ | | |
| 9 | NY1 | | | | | | ○ | ○ |
| 10 | NY8 | | | ○ | ○ | | | |
| 11 | NY11 | | | | | | | |
| 12 | NY31 | | ○ | ○ | ○ | ○ | | |
| 13 | 26695 | | | | | | | ○ |
| 14 | HPK5 | | | ○ | ○ | | ○ | ○ |
| 15 | HPKT510 | ○ | | | | ○ | | |
| 16 | 11637 | ○ | ○ | | ○ | | | |

**[Table 13]**

| Total mineral concentration = 3,500 mg/kg | | | | | | | | |
|---|---|---|---|---|---|---|---|---|
| No. | Bacteria name | A | B | C | D | E | F | G |
| 1 | KMT27 | ○ | ○ | ○ | ○ | ○ | ○ | ○ |
| 2 | KMT31 | ○ | ○ | ○ | ○ | ○ | ○ | ○ |
| 3 | KMT40 | ○ | ○ | ○ | ○ | ○ | ○ | ○ |
| 4 | KMT44 | ○ | ○ | ○ | ○ | ○ | ○ | ○ |
| 5 | KMT45 | ○ | ○ | ○ | ○ | ○ | ○ | ○ |
| 6 | KMT46 | ○ | ○ | ○ | ○ | ○ | ○ | ○ |
| 7 | KMT47 | ○ | ○ | ○ | ○ | ○ | ○ | ○ |
| 8 | KMT50 | ○ | ○ | ○ | ○ | ○ | ○ | ○ |
| 9 | NY1 | ○ | ○ | ○ | ○ | ○ | ○ | ○ |
| 10 | NY8 | | | ○ | ○ | | | |
| 11 | NY11 | ○ | | | | ○ | ○ | ○ |
| 12 | NY31 | | ○ | ○ | ○ | ○ | | |
| 13 | 26695 | | | | | | | |
| 14 | HPK5 | | | ○ | ○ | | ○ | ○ |
| 15 | HPKT510 | ○ | | | | ○ | | |
| 16 | 11637 | ○ | ○ | | ○ | | | |

**[Table 14]**

| Total mineral concentration = 5,900 mg/kg | | | | | | | | |
|---|---|---|---|---|---|---|---|---|
| No. | Bacteria name | A | B | C | D | E | F | G |
| 1 | KMT27 | ○ | ○ | ○ | ○ | ○ | ○ | ○ |
| 2 | KMT31 | ○ | ○ | ○ | ○ | ○ | ○ | ○ |
| 3 | KMT40 | ○ | ○ | ○ | ○ | ○ | ○ | ○ |
| 4 | KMT44 | ○ | ○ | ○ | ○ | ○ | ○ | ○ |
| 5 | KMT45 | ○ | ○ | ○ | ○ | ○ | ○ | ○ |
| 6 | KMT46 | ○ | ○ | ○ | ○ | ○ | ○ | ○ |
| 7 | KMT47 | ○ | ○ | ○ | ○ | ○ | ○ | ○ |
| 8 | KMT50 | ○ | ○ | ○ | ○ | ○ | ○ | ○ |
| 9 | NY1 | ○ | ○ | ○ | ○ | ○ | ○ | ○ |
| 10 | NY8 | ○ | ○ | ○ | ○ | ○ | ○ | ○ |
| 11 | NY11 | ○ | ○ | ○ | ○ | ○ | ○ | ○ |
| 12 | NY31 | ○ | ○ | ○ | ○ | ○ | ○ | ○ |
| 13 | 26695 | ○ | ○ | ○ | ○ | ○ | ○ | ○ |
| 14 | HPK5 | ○ | ○ | ○ | ○ | ○ | ○ | ○ |
| 15 | HPKT510 | ○ | ○ | ○ | ○ | ○ | ○ | ○ |
| 16 | 11637 | ○ | ○ | ○ | ○ | ○ | ○ | ○ |

**[Table 15]**

| Total mineral concentration = 11,800 mg/kg | | | | | | | | |
|---|---|---|---|---|---|---|---|---|
| No. | Bacteria name | A | B | C | D | E | F | G |
| 1 | KMT27 | ○ | ○ | ○ | ○ | ○ | ○ | ○ |
| 2 | KMT31 | ○ | ○ | ○ | ○ | ○ | ○ | ○ |
| 3 | KMT40 | ○ | ○ | ○ | ○ | ○ | ○ | ○ |
| 4 | KMT44 | ○ | ○ | ○ | ○ | ○ | ○ | ○ |
| 5 | KMT45 | ○ | ○ | ○ | ○ | ○ | ○ | ○ |
| 6 | KMT46 | ○ | ○ | ○ | ○ | ○ | ○ | ○ |
| 7 | KMT47 | ○ | ○ | ○ | ○ | ○ | ○ | ○ |
| 8 | KMT50 | ○ | ○ | ○ | ○ | ○ | ○ | ○ |
| 9 | NY1 | ○ | ○ | ○ | ○ | ○ | ○ | ○ |
| 10 | NY8 | ○ | ○ | ○ | ○ | ○ | ○ | ○ |
| 11 | NY11 | ○ | ○ | ○ | ○ | ○ | ○ | ○ |
| 12 | NY31 | ○ | ○ | ○ | ○ | ○ | ○ | ○ |
| 13 | 26695 | ○ | ○ | ○ | ○ | ○ | ○ | ○ |
| 14 | HPK5 | ○ | ○ | ○ | ○ | ○ | ○ | ○ |
| 15 | HPKT510 | ○ | ○ | ○ | ○ | ○ | ○ | ○ |
| 16 | 11637 | ○ | ○ | ○ | ○ | ○ | ○ | ○ |

Increasing the total mineral concentration in mineral-rich waters prepared from deep sea water significantly increased the types of bacterial strains whose proliferation could be inhibited, and once the total mineral concentration reached 5,900 mg/kg, these mineral-rich waters were found effective in inhibiting the proliferation of all types of Helicobacter pylori strains. In addition, the effects of inhibiting the proliferation of Helicobacter pylori strains became particularly prominent when the magnesium concentration was high.

## Claims

1. An agent for use in the treatment of diseases of the upper gastrointestinal tracts, autoimmune diseases and acute coronary diseases, wherein the diseases are caused by Helicobacter pylori strains, **characterized in that** the agent is mineral-rich water derived from deep sea water, and contains as minerals at least one or more of magnesium, calcium, sodium and potassium, wherein the mineral content per kilogram is 5,900 mg or more.

## Patentansprüche

1. Ein Mittel zur Verwendung bei der Behandlung von Erkrankungen des oberen Gastrointestinaltrakts, Autoimmunerkrankungen und akuten Koronarerkrankungen, wobei die Erkrankungen durch Heliobacter Pylori-Stämme verursacht werden, **dadurch gekennzeichnet, dass** das Mittel ein von Tiefseewasser abgeleitetes mineralreiches Wasser ist und als Mineralien mindestens ein oder mehrere von Magnesium, Calcium, Natrium und Kalium enthält, wobei der Mineralgehalt pro Kilogramm bei 5900 mg oder mehr liegt.

## Revendications

1. Agent pour une utilisation dans le traitement de maladies du tractus gastrointestinal supérieur, de maladies auto-immunes et de maladies coronariennes aigues, dans lesquelles les maladies sont causées par la souche bactérienne Helicobacter pylori, **caractérisé en ce que** l'agent est une eau riche en minéraux dérivée d'eau de haute mer, et contient comme minéraux au moins un ou plusieurs parmi le magnésium, le calcium, le sodium et le potassium, dans lequel le contenu en minéraux est de 5900 mg ou plus par kilogramme.
